# EUROPEAN PATENT APPLICATION

(11) **EP 1 470 802 A1**
(43) Date of publication of application: **27.10.2004**
(21) Application number: 04251871.2
(22) Date of filing: 30.03.2004
(51) Int. Cl.: A61F 2/40

(54) **Articulating surface replacement prosthesis**

(30) Priority: 31.03.2003 US 403750
(71) Applicant: Depuy Products, Inc., Warsaw, Indiana 46581 (US)
(72) Inventor: Maroney, Brian J., Ft. Wayne, Indiana (US); Long, Jack F., Warsaw, Indiana (US); Iannotti, Joseph P., Maryfield Heights, Ohio (US); Williams, Gerald R., Jr., Vilanova, Philadelphia (US)
(74) Representative: Belcher, Simon James

(57) **Abstract**

A prosthesis (10) for fitting to a long bone joint arthroplasty includes a body (20) having an articulating surface (22) and a support surface (34) opposed to the articulating surface (22). The support surface (34) is adapted for intimate contact with a prepared surface (18) of the long bone (4).

## Description

The present invention relates generally to the field of orthopaedics, and more particularly, to an implant for use in arthroplasty.

The invention relates to implantable articles and methods for implanting such articles. More particularly, the invention relates to a bone prosthesis and a method for implanting the same.

There are known to exist many designs for and methods for implanting implantable articles, such as bone prostheses. Such bone prostheses include components of artificial joints, such as elbows, hips, knees and shoulders. An important consideration in the design and implanting of virtually any implantable bone prosthesis is that the bone have adequate fixation when implanted within the body.

Earlier designs of implantable articles relied upon the use of cement, such as polymethylmethacrylate (PMMA) to anchor the implant. The use of such implants can have some advantages, such as providing a fixation that does not develop free play or does not lead to erosion of joining faces postoperatively. However, the current trend is to use the cements to a lesser extent because of their tendency to lose adhesive properties over time and the possibility that cement contributes to wear debris within a joint.

Recently, implantable bone prostheses have been designed such that they encourage the growth of hard bone tissue around the implant. Such implants are often implanted without cement and the bone grows around surface irregularities, for example, porous structures on the implant.

One such implantable prosthesis is a shoulder prosthesis. During the lifetime of a patient, it may be necessary to replace the natural humeral head and associated glenoid cavity with a prosthesis. Such a shoulder replacement procedure may be necessary to be performed on a patient as a result of, for example, disease or trauma, for example, disease from osteoarthritis or rheumatoid arthritis.

Most shoulder replacement surgeries today involve the implantation of a total shoulder prosthesis. In a total shoulder replacement procedure, a humeral component having a head portion is utilized to replace the natural head portion of the upper arm bone or humerus. The humeral component typically has an elongated intramedullary stem which is utilized to secure the humeral component to the patient's humerus. In such a total shoulder replacement procedure, the natural glenoid surface of the scapula is restructured or otherwise replaced with a glenoid component that provides a bearing surface for the head portion of the humeral component.

With the average age of patients requiring shoulder arthroplasty decreasing, orthopaedic implant manufacturers are developing "bone-sparing" implants for the initial treatment of degenerative arthritis. While bone-sparing implants for the treatment of hip and knee arthroplasty are becoming quite common, bone-sparing shoulder arthroplasty techniques and prostheses are also being developed.

Shoulder surface replacement prostheses are being developed to replace the articulating surface of the proximal humerus with a minimal bone resection and minimal disruption of the metaphysis and the diaphysis. Current designs use a semi-spherical articular dome with a small stem for rotational stability. The under surface of the articular head is also semi-spherical and meets with a spherically machined humeral head.

Typically, however, arthritis of the gleno-humeral joint causes flattening of the humeral head with a large medial osteophyte. The flat humeral head can cause voids in the bone under the prosthesis resulting in limited contact between the prosthesis and the resected bone and may limit the load transfer capability between the prosthesis and the humerus.

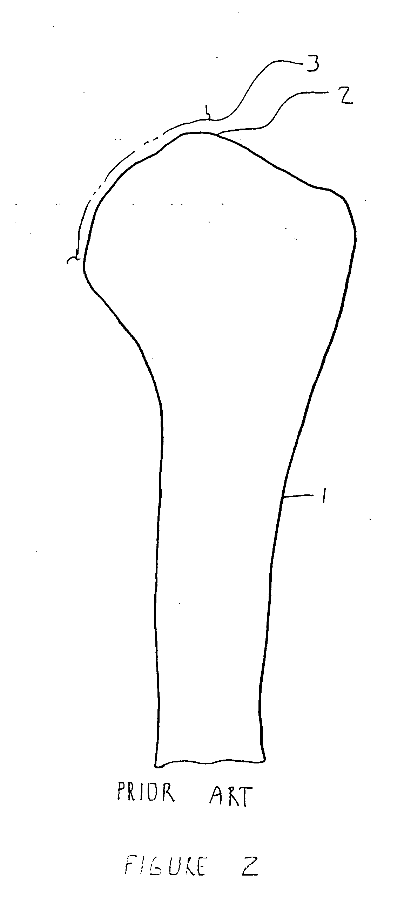

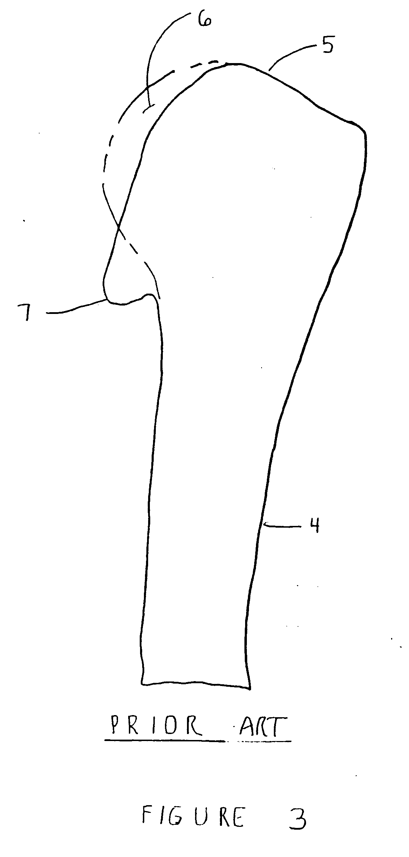

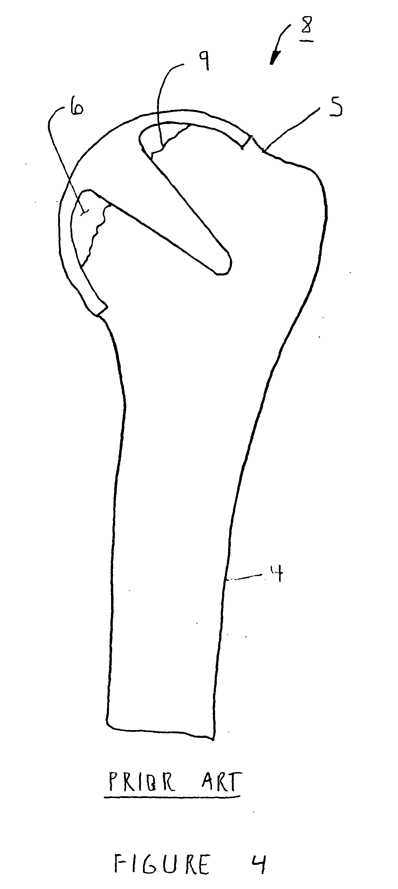

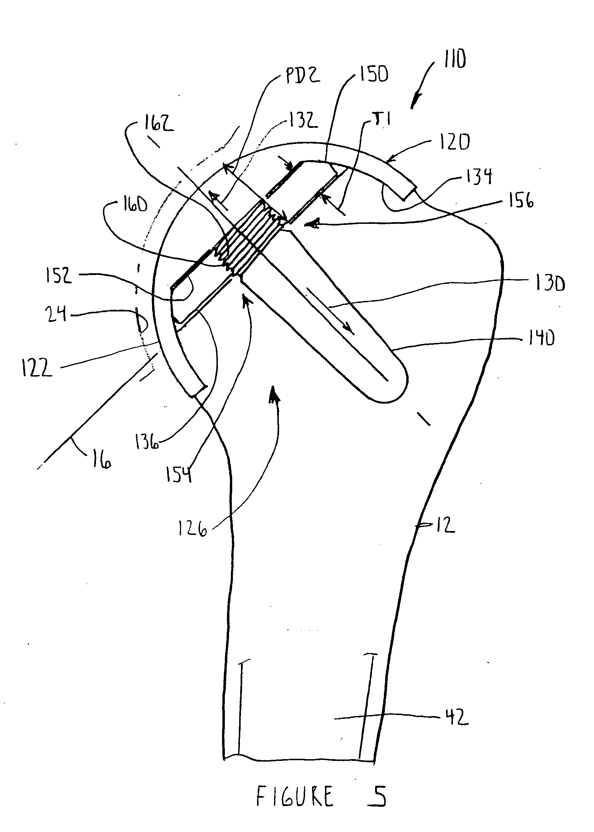

Referring now to FIGURE 2, a healthy long bone or, in the form of, for example, a humerus 1 is shown. The humerus 1 includes a head 2 on the proximal end of the humerus 1. The head 2 of a healthy humerus has an arcuate outer periphery. The arcuate outer periphery is generally hemispherical and meets with a concave glenoid cavity 3.

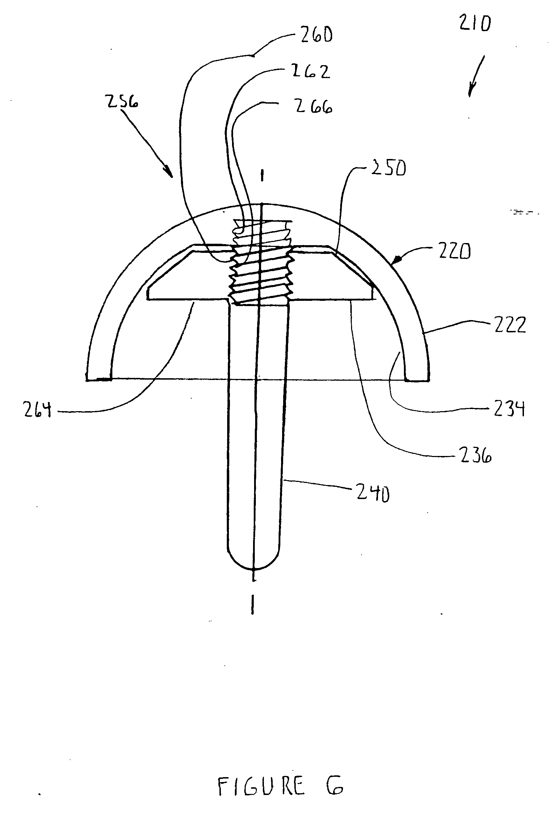

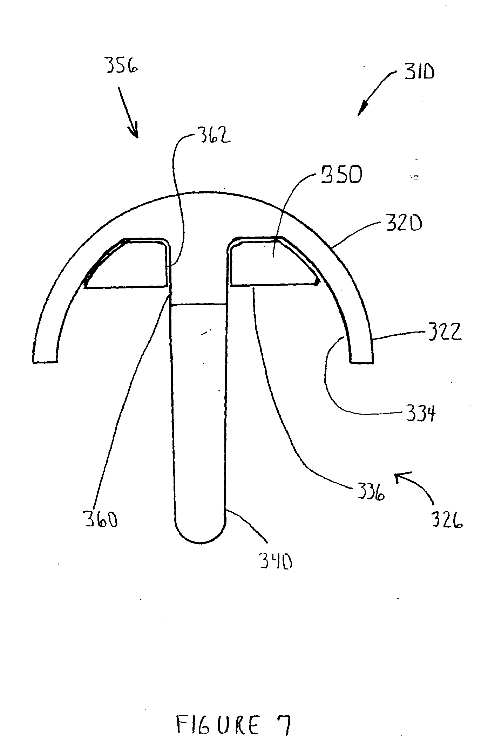

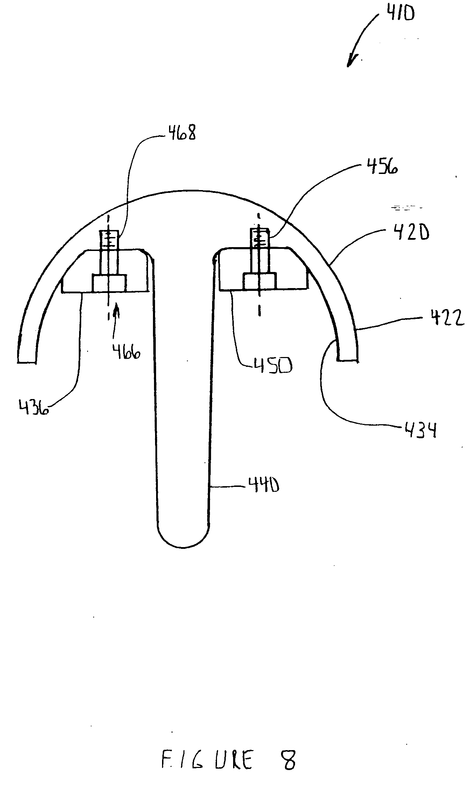

Referring now to FIGURE 3, a diseased humerus 4 is shown. The diseased humerus 4 includes a head 5. The head 5 is flattened as shown in FIGURE 3. The humerus 4 also has developed a large medial osteophyte 7.

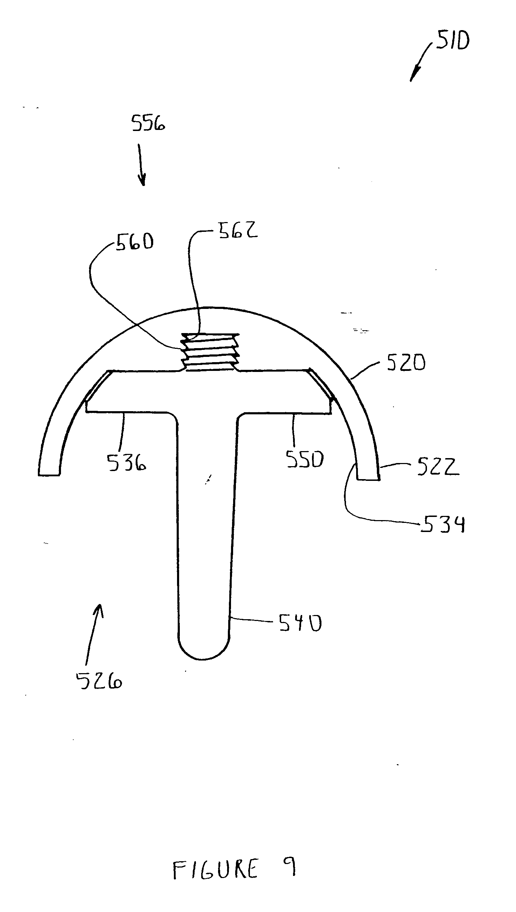

Referring now to FIGURE 4, a prior art prosthesis 8 is shown in position on the head 5 of diseased humerus 4. The head 5 includes a flattened humeral head area or bony defect 9, which leads to a void 6 between the prosthesis 8 and the bony defect 9.

The present invention provides for a humeral surface replacement prosthesis, which provides for support between the prosthesis and the flattened natural humeral head. The humeral head replacement prosthesis includes a support surface, which is in engagement with the bone surface under the prosthesis.

According to one embodiment of the present invention, there is provided a prosthesis for use in performing joint arthroplasty. The prosthesis is to be fitted to a long bone. The prosthesis includes a body having an articulating surface and a support surface opposed to the articulating surface. The support surface is adapted for intimate contact with a prepared surface of the long bone.

According to another embodiment of the present invention, a kit is provided for use in performing joint arthroplasty on a bone. The kit includes a prosthetic member having an articulating surface and a support surface opposed to the articulating surface. The kit also includes a first spacer and a second spacer. The first spacer may be positioned between the long bone and the support surface of the prosthetic member. The second spacer may be positioned between the long bone and the support surface of the prosthetic member. The prosthetic member and at least the first spacer or the second spacer may be used selectively to form a properly sized prosthesis to perform the joint arthroplasty.

According to a further embodiment of the present invention, a method is provided for joint arthroplasty. The method includes the steps of providing a prosthetic member, providing a plurality of spacers, making a measurement of the contour of a long bone, selecting one of the plurality of spacers based upon the measurement of the contour, and implanting the prosthetic member and the selected one of the plurality of spacers onto the long bone. The technical advantages of the present invention include an increased load contact area. For example, according to one aspect of the present invention, the prosthesis of the present invention includes a body, which has a support surface, adapted for intimate contact with a surface of a long bone prepared to remove a bony defect. Thus, the present invention provides for increased load contact area by eliminating the void otherwise experienced in such an application.

Another technical advantage of the present invention includes increased bonding surface contact. For example, according to another aspect of the present invention, the prosthesis includes a body having a support surface adapted for intimate contact with a surface of the long bone prepared to remove a bony defect. Thus the support surface is in full contact with the resected prosthesis. Thus, the present invention provides for increased bonding surface contact.

Another technical advantage of the present invention includes the ability to accommodate different amounts of defect. According to one aspect of the present invention, a kit is provided which includes a prosthetic member with a support surface opposed to the articulating surface and a first spacer positioned between the long bone and the support surface, as well as a second spacer of different thickness, which may also be positioned against the support surface. Thus, the present invention is able to accommodate patients, which have a humerus with a different amount of defect or void.

The technical advantages of the present invention further include the ability to accommodate a wide range of patients. For example, according to another aspect of the present invention, the prosthesis may include a body as well as a spacer and/or a stem, which may be removably secured to the prosthesis. By selecting one of a plurality of bodies, spacers and stems, a multitude of prosthetic assemblies may be provided with a minimal amount of parts.

A further technical advantage of the present invention includes the ability to reduce the amount of inventory required by an orthopaedic manufacturer or a hospital. For example, according to one aspect of the present invention, a plurality of prostheses can be made from a kit of a plurality of bodies, spacers and stems, one of each which may be joined to form a prosthetic assembly. By selecting from the variety of bodies, spacers and stems a large number of prostheses can be provided with minimal inventory.

The prosthesis of the present invention can be used in a method for providing joint arthroplasty comprising:
providing a prosthetic member;
providing a plurality of spacers;
making a measurement of the contour of a long bone;
selecting one of the plurality of spacers based upon the measurement of the contour; and
implanting the prosthetic member and the selected one of the plurality of spacers onto the long bone.

Embodiments of the invention will now be described by way of example with reference to the accompanying drawings, in which:
FIGURE 1 is a plan view partially in cross section of a surface replacement prosthesis according to the present invention for use on a diseased humerus including a planar support surface;
FIGURE 2 is a plan view of a healthy humerus;
FIGURE 3 is a plan view of a diseased humerus;
FIGURE 4 is a plan view partially in cross section of a prior art humeral prosthesis;
FIGURE 5 is a plan view partially in cross section of another embodiment of a surface replacement prosthesis according to the present invention for use on a diseased humerus including a planar support surface;
FIGURE 6 is a plan view partially in cross section of the prosthesis of Figure 5;
FIGURE 7 is a plan view partially in cross section of another embodiment of a surface replacement prosthesis according to the present invention including a spacer and a hemispherical cup having a tapered stem;
FIGURE 8 is a plan view partially in cross section of another embodiment of a surface replacement prosthesis according to the present invention including an integral prosthesis and stem with a spacer bolted to the prosthesis;
FIGURE 9 is a plan view partially in cross section of another embodiment of a surface replacement prosthesis according to the present invention including an integral stem and spacer threaded to the prosthesis;
FIGURE 10 is a plan view partially in cross section of another embodiment of a surface replacement prosthesis according to the present invention including an integral cup and stem with a spacer threaded to the cup and having a porous coating on the spacer and the prosthesis;
FIGURE 11 is a plan view partially in cross section of a surface replacement prosthesis including a modular prosthesis including a stem and spacer secured to the prosthesis with a tapered connection and with the prosthesis and having a porous coating on the spacer and the stem;
FIGURE 12 is an exploded plan view partially in cross section of another embodiment of a surface replacement prosthesis according to the present invention including a modular prosthesis including a hemispherical cup and a plug, the prosthesis also including two spacers and a separate stem, with the components being interconnected with a tapered connection;
FIGURE 13 is a plan view partially in cross section of another embodiment of a surface replacement prosthesis according to the present invention including a hemispherical cup, a separate spacer and a separate stem with the components being interconnected with a tapered connection;
FIGURE 14 is a plan view of kit for use in performing shoulder arthroplasty surgery according to a further embodiment of the present invention;
FIGURE 15 is a plan view partially in cross section of a gauge for determining the appropriate spacer for use with the surface replacement prosthesis of the present invention for use on a diseased humerus;
FIGURE 16 is a guide pin for use with the present invention;
FIGURE 17 is a perspective view of a trial for use in performing shoulder arthroplasty surgery with the prosthesis according to a further embodiment of the present invention;
FIGURE 18 is a plan view of the trial of FIGURE 17;
FIGURE 19 is a plan view of a set of instruments including gauges for use in performing shoulder arthroplasty surgery according to a further embodiment of the present invention;
FIGURE 20 is plan view of a cutter used to prepare a humerus for implantation of a prosthesis according to the present invention; and
FIGURE 21 is a flow chart of a method of performing arthroplasty according to the present invention; and
FIGURE 22 is a plan view, partially in cross section, of a hip prosthesis for use in a femur according to a further embodiment of the present invention.

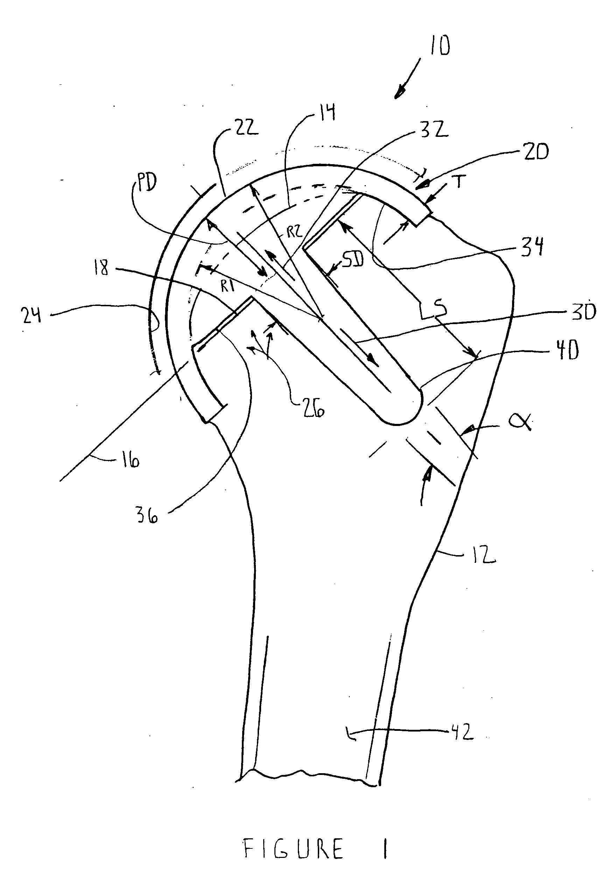

Referring to the drawings, FIGURE 1 shows a prosthesis 10 according to the present invention is shown. The prosthesis 10 is used in performing joint arthroplasty. For example, the prosthesis 10 may be used for hip or shoulder arthroplasty.

As shown in FIGURE 1, the prosthesis 10 is shown for use with a long bone 12 in the form of a humerus. It should be appreciated that the prosthesis 10 may be suited for another long bone, for example, the femur.

Typically, when the prosthesis 10 is required on a humerus 12, the humerus 12 includes a flattened humeral head forming a bony defect 14. Preferably and as shown in FIGURE 1, the humerus 12 is resected along resection plane 16 providing a prepared surface 18. The prosthesis 10 includes a body 20. The body 20 has an articulating surface 22, which is in a combination of mostly rolling and sliding contact with glenoid cavity 24. The body 20 also includes a support surface 26 opposed to the articulating surface 22. As can be seen in FIGURE 1, the support surface 26 is located in a first direction 30 opposed to the second direction 32 of the articulating surface 22.

The prosthesis 10 may have any suitable size and shape capable of providing the articulating surface 22 for cooperation with the glenoid cavity 24 and to provide the intimate contact with the resected surface 18 of the humerus 12.

As shown in FIGURE 1, the body 20 may have a shape such that articulating surface 22 is convex. Opposed from the articulating surface 22 may be an arcuate support surface 34. The arcuate support surface 34 may be concave. For example, the arcuate support surface 34 and the articulating surface 22 may form a sector of a hollow sphere having a thickness T and with the articulating surface 22 being defined by a radius R2 and the arcuate support surface 34 being defined by a radius R1.

For simplicity and as shown in FIGURE 1, the support surface 26 may include a planar portion 36 which, depending on the position of the resected plane 16, may be defined by a plane dimension PD. The planar surface 36 and the arcuate support surface 34 define the support surface 26. With the planar support surface 36 and the resected surface 18 both being planar, the resected surface 18 and the planar portion 36 provide intimate contact between the prosthesis 10 and the humerus 12.

For sufficient securement of the prosthesis 10 to the humerus 12, the prosthesis 10 may further include a stem 40 for securement with the cancellous bone 42 of the humerus 12. The stem 40 may have any suitable size and shape capable of securing the prosthesis 10 to the humerus 12. For example, the stem 40 may have a generally cylindrical shape defined by diameter SD and may have a length LS extending distally from the planar portion 36 in the first direction 30. The stem 40, as shown in FIGURE 1, may have a slight taper defined by angle α. For example, the angle α may be 30 to 20°.

As shown in FIGURE 1, the prosthesis 10 may be integral or made from a single piece. The prosthesis 10 may be made of any suitable durable material that is compatible with the human anatomy and provides sufficient strength and wear properties. For example, the prosthesis 10 may be made of a durable plastic, a ceramic or a metal. For durability and strength, the prosthesis 10 is preferably made of a metal. For example, the prosthesis 10 may be made of a titanium alloy, a cobalt chromium alloy, or stainless steel.

The prosthesis 10 may be made by any suitable process. For example, the prosthesis 10 may be machined from bar stock, forged, cast or made from a material such as that available from Liquidmetal Technologies, 100 North Tampa St., Suite 3150, Tampa, Florida 33602.

It should be appreciated that due to the variations in the size of the patient and his or her respective humerus, a wide variety of prosthesis 10 may be required to accommodate the variations in a patient's humerus. Not only may the selection of the proper prosthesis 10 be governed by the proper radius R2 of the articulating surface 22, variations in the progress of the osteoarthritis may result in a bony defect 14 being of various stages of progression. Due to the changes in the progression of the disease and the resulting shape of the bony defect 14, the resection plane 16 may vary from being somewhat shallow to being much deeper into the humerus 12. Therefore, even for a given size of the articulating surface 22, the position of the planar portion 36 of the support surface 26 may vary. These various needs may be accomplished by providing a wide variety of size and configurations of the prosthesis 10. The applicants have discovered that the prosthesis may be made with more than one component.

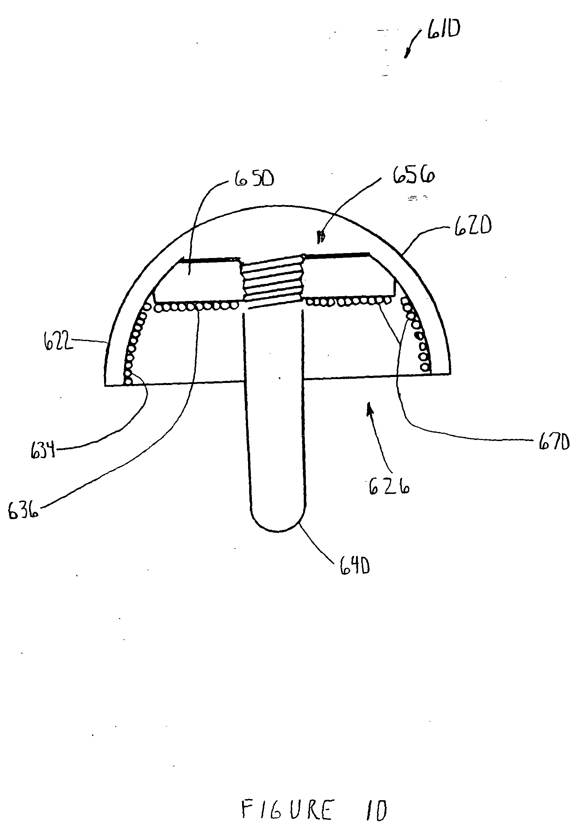

Referring now to FIGURE 5, an example of a multi-piece prosthesis is shown as prosthesis 110. Prosthesis 110 of FIGURE 5 is similar to the prosthesis 10 of FIGURE 1 except that the prosthesis 110 is made of two components rather than the solitary component of the prosthesis 10 of FIGURE 1.

As shown in FIGURE 5, the prosthesis 110 includes in addition to body 120, a spacer 150. The spacer 150 provides for a variety of locations of planar portion 136 of support surface 126. Thus, by utilizing the prosthesis 110, a common body 120 may be used with a variety of spacers 150 having different thicknesses T1. Thus, for any prosthesis 110 a plurality of planar dimensions PD2 may be provided by merely changing the spacer 150 to either a thinner or a thicker spacer.

As shown in FIGURE 5, the prosthesis 110 includes the body 120. The body 120 is similar to the body 20 of the prosthesis 10 of FIGURE 1 and includes an articulating surface 122 extending in a second direction 132 as well as a stem 140 extending in a first direction 130 opposed to the second direction 132. The stem 140 is similar stem 40 of the prosthesis 10.

As shown in FIGURE 5, the body 120 includes a body planar surface 152 to which the spacer 150 is placed. The spacer 150 defines the planar portion 136 of the support surface 126 and works in conjunction with arcuate surface 134 of the body 120 to support the prosthesis 110 against the humerus wall.

As shown in FIGURE 5, the spacer 150 preferably has a pair of spaced apart parallel faces defined with the thickness T1. The spacer 150 has a central opening 154 to permit the spacer 150 to be positioned in place against the body planar surface with the stem 140 passing through the opening 154.

Preferably, and as shown in FIGURE 5, the spacer 150 is secured to the body 120 by, for example, a connector 156. The connector 156 may, as shown in FIGURE 5, be in the form of a threadable connection. For example, the connector 156 may include external threads 160 located on the stem 140. The external threads 160 on the stem 140 cooperate with matching internal threads 162 on the spacer 150. A feature (not shown) in the form of, for example, a recess on the planar portion 136 of the spacer 150 may be utilized to secure the spacer 150 against the body 120.

The body 120 and the spacer 150 may be made of a similar material to that of the body 20 of the prosthesis 10. Thus, for example, the body 120 and the spacer 150 may be made of a cobalt chromium alloy, a titanium alloy or a stainless steel alloy.

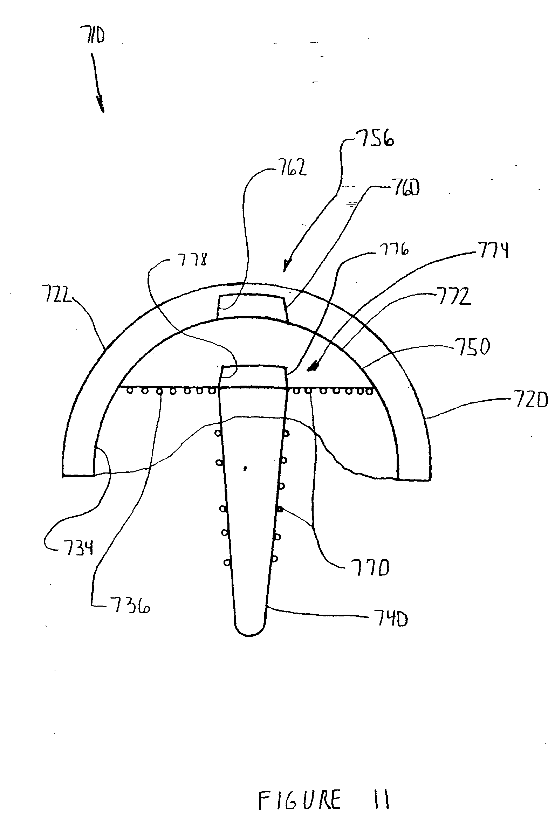

Referring now to FIGURE 6, another embodiment of a multi-piece prosthesis is shown as prosthesis 210. The prosthesis 210 is similar to the prosthesis 110 of FIGURE 5 and includes a body 220 as well as a spacer 250. The prosthesis 210 further includes a stem 240. The body 220 of the prosthesis 210 is different than the body 120 of the prosthesis 110 in that the body 220 does not include the stem 240. In the prosthesis 210 of FIGURE 6, the stem 240 is a separate component.

As shown in FIGURE 6, the spacer 250 is contained between the stem 240 and the prosthesis 210. The body 220 as shown in FIGURE 6 has a generally hollow hemispherical shape having a convex outer articulating surface 222 and a convex arcuate support surface 234. A first connector 256 is used to secure the stem 240 to the body 220. The connector 256 may as shown in FIGURE 6 be in the form internal threads 262 in the body 220 which mate with corresponding external threads 260 on the stem 240. A second connector 264 is used to secure the spacer 250 to the stem 240. The second connector 264 may be in the form internal threads 266 on the spacer 250 which mate with the external threads 260 on the stem 240. It should be appreciated that alternatively the second connector 264 may be in the form of a shoulder extending from the outer periphery of the stem 240 which mates with the planar portion 236 of the spacer 250.

Referring now to FIGURE 7, another embodiment of the present invention is prosthesis 310. Prosthesis 310 includes a body 320 similar to the body 120 of the prosthesis 110 of FIGURE 5 in that the body 320 includes stem 340 similar to stem 140 of FIGURE 5. The prosthesis 310 further includes a spacer 350 similar to the spacer 150 of the prosthesis 110 of FIGURE 5.

The spacer 350 is secured to the body 320 by means of a connector 356. The connector 356 is different that the connector 156 of the prosthesis 110 in that the connector 356 is in the form of a taper fit. The spacer 350 includes a tapered opening 362, which engages with tapered stem portion 360 of the stem 340 of the prosthesis 310. The body 320 includes an articulating surface 322 and an opposed arcuate support surface 334. The spacer 350 includes a planar support surface 336 that together with the arcuate support surface 334 form support surface 326 for supporting the prosthesis 310 within the humerus 12.

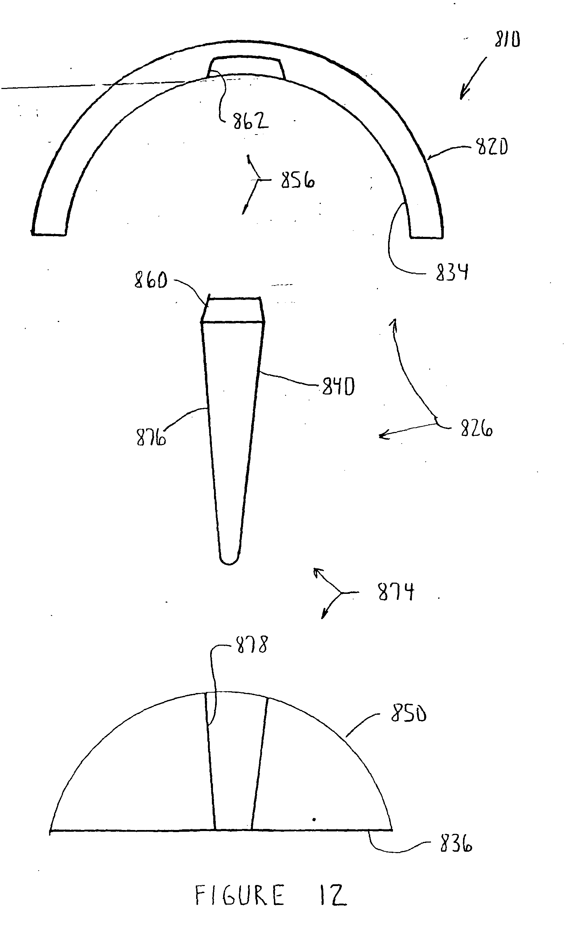

Referring now to FIGURE 8, another embodiment of the present invention is shown as prosthesis 410. The prosthesis 410 of FIGURE 8 is similar to the prosthesis 310 of FIGURE 7, and includes a body 420 similar to the body 320 of FIGURE 7. The body 420 includes an articulating surface 422 and an opposed arcuate support surface 434. The body 420 in integral with a stem 440 similar to the stem 340 of FIGURE 7. The prosthesis 410 further includes a spacer 450 similar to the spacer 350 of the prosthesis 310 of FIGURE 7.

The spacer 450 is secured to the body 420 of the prosthesis 410 by means of a connector 456, which is different than the connector 356 of the prosthesis 310 of FIGURE 7. The connector 456 is in the form of a plurality of socket head hex cap screws. The cap screws 456 are fitted through recessed openings 466 in the spacer 450. The cap screws 456 are secured to the body 420 by a plurality of threaded openings 468. The spacer 450 provides planar support surface 436.

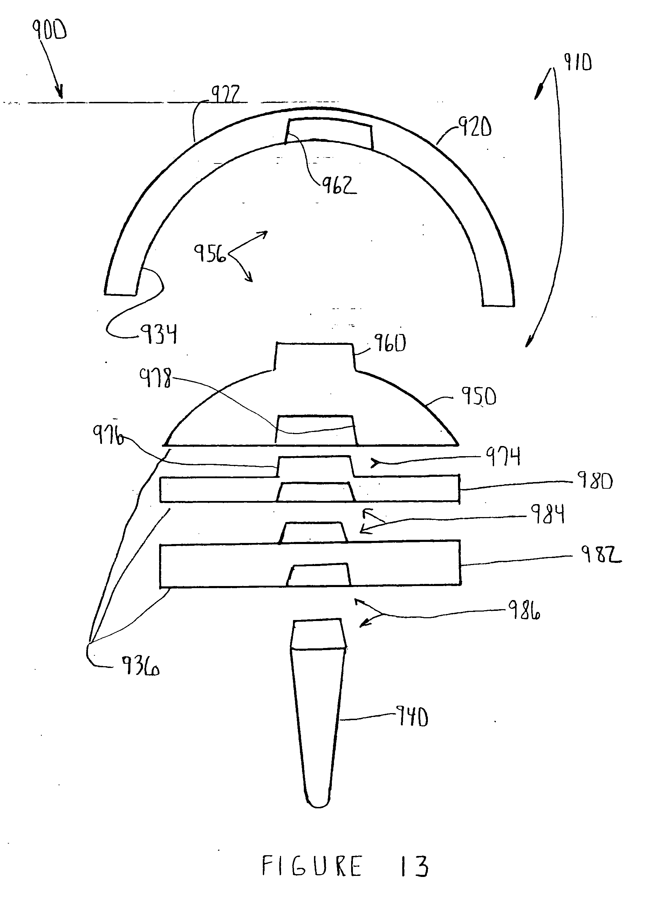

Referring now to FIGURE 9, another embodiment of the present invention is shown as prosthesis 510. Prosthesis 510 is similar to the prosthesis 110, 210, 310 and 410 in that the prosthesis 510 includes a body 520, a spacer 550, and a stem 540. The prosthesis 510 is different than the prosthesis 110, 210, 310 and 410 in that the spacer 550 and the stem 540 are integral with each other. The body 520 of the prosthesis 510 thus does not include the stem 540 and is a separate part from the spacer 550 and the stem 540.

As shown in FIGURE 9, the body 520 has a generally hollow hemispherical shape having a articulating surface 522 and an opposed arcuate support surface 534. The spacer 550 has a general disc shape with the stem 540 having a generally cylindrical shape and extending outwardly from the centre portion of the spacer 550. The spacer 550 is secured to the body 520 by means of a connector 556.

The connector 556 as shown in FIGURE 9 is in the form of a threaded stem extending from the spacer 550 in a direction opposed to the stem 540. The connector 556 includes external threads 560, which mate with internal threads 562 in the body 520. The spacer 550 forms planar support surface 536, which together with the arcuate support surface 534, forms support surface 526 for supporting the prosthesis 510 against the humerus 12.

Referring now to FIGURE 10, another embodiment of the present invention is shown as prosthesis 610. Prosthesis 610 is similar to the prosthesis 110 of FIGURE 5. Prosthesis 610 includes a body 620 similar to the body 120 of FIGURE 5 and includes an articulating surface 622 and opposed arcuate support surface 634. The body 620 includes a stem 640 similar to the stem 140 of FIGURE 5. The prosthesis 610 further includes a spacer 650 similar to the spacer 150 of FIGURE 5. The spacer 650 includes a planar support surface 636, which together with the arcuate support surface 634 serve to form support surface 626 for supporting the prosthesis 610 against the humerus 12. The prosthesis 610 further includes a connector 656 similar to the connector 156 of the prosthesis 110 of FIGURE 5.

Unlike the prosthesis 110, the prosthesis 610 includes a porous coating 670 located on the planar support surface 636 and the arcuate support surface 634. The porous coating 670 serves to provide additional surface for promoting bony ingrowth into the prosthesis 610 for improved fixation of the prosthesis 610 to the humerus 12. Any suitable commercially available porous coating may be suitable for the coating 670. Products having appropriate porous coating are available from DePuy Orthopaedics Inc under the trade mark POROCOAT. Features of such coatings are disclosed in US-3855638.

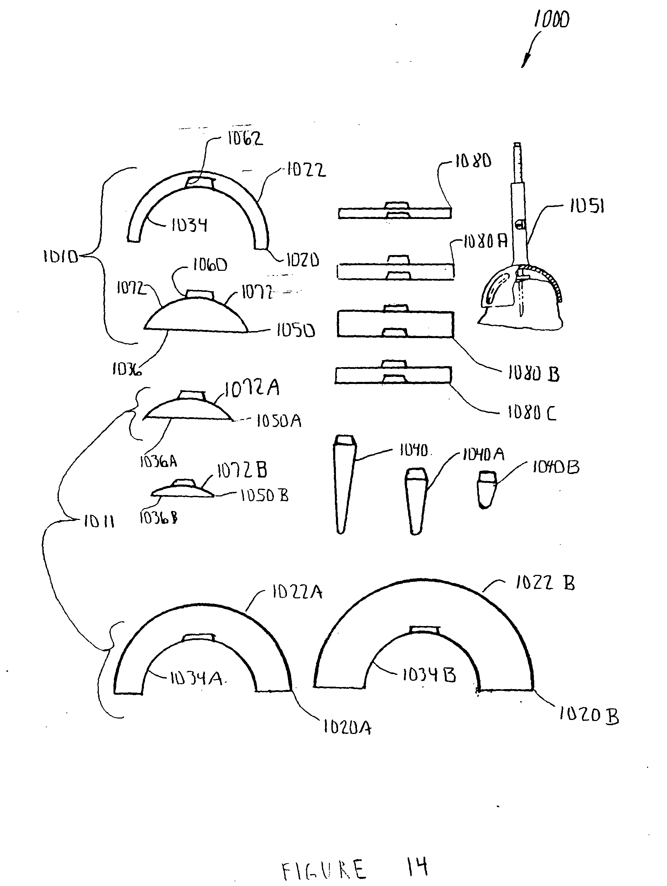

Referring now to FIGURE 11, another embodiment of the present invention is shown as prosthesis 710. Prosthesis 710 is a three-part prosthesis including a body 720 similar to the body 220 of the prosthesis 210 of FIGURE 6. The body 720 includes a hemispherical outer articulating surface 722 and a concave internal arcuate support surface 734.

The prosthesis 710 further includes a plug 750, which serves the purpose of the spacer 250 of the prosthesis 210 of FIGURE 6. The plug 750 includes a planar support surface 736 and an opposed spherical outer surface 772 which mates with the arcuate support surface 734 of the body 720. The plug 750 may be secured to the body 720 by any suitable method. For example, as shown in FIGURE 11, a first connector 756 in the form a taper connection is shown.

The first connector 756 includes an external taper 760 extending from the plug 750, which mates with an internal taper 762 in the body 720. The prosthesis 710 further includes a generally cylindrical tapered stem 740, which is secured to the plug 750 by a second connector 774.

The stem 740 may be secured to the plug 750 by, for example, the second connector 774. The second connector 774 may have any suitable configuration and may, as shown in FIGURE 11, be in the form of an external taper 776 located on the stem 740, which cooperates with an internal taper 778 formed in the plug 750.

As shown in FIGURE 11, the prosthesis 710 may further include a coating 770 in the form of, for example, a porous coating, for example of the kind discussed above and disclosed in US-3855638 to encourage ingrowth to assist in securing the prosthesis 710 to the humerus 12. The coating 770 may be secured to the stem 740 as well as to the arcuate support surface 734 as well as the planar support surface 736.

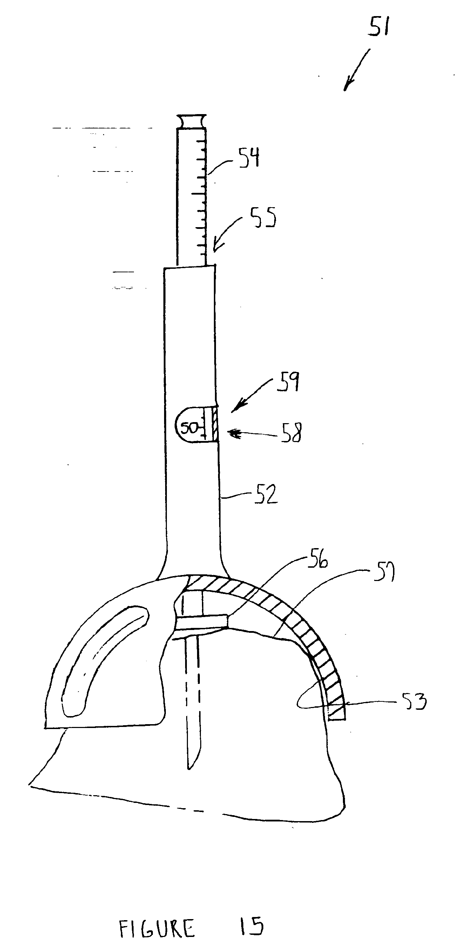

Referring now to FIGURE 12, another embodiment of the present invention is shown as prosthesis 810. Prosthesis 810 is similar to prosthesis 710 of FIGURE 11 and includes three components, namely a body 820 similar to body 720 of the prosthesis 710 of FIGURE 11, a stem 840 similar to the stem 740 of the prosthesis 710 of FIGURE 11, and a plug 850. The plug 850 is similar to the plug 750 of the prosthesis 710 of FIGURE 11 except that the plug 850 and the stem 840 are secured to the body 820 in a different fashion from that of the prosthesis 710.

While the prosthesis 810 similar to the prosthesis 710 has its components interconnected by means of tapered connections, the tapered connections of the prosthesis 810 are different from those of the prosthesis 710 of FIGURE 11. For example, the prosthesis 810 includes a first connector 856 in the form a tapered connection. The tapered connection 856 includes an external taper 860 formed on the stem 840 which connects with an internal taper 862 formed on the body 820.

The plug 850 is secured to the stem 840 by means of a second tapered connection 874. The second tapered connection 874 includes an external taper 876 formed on the stem 840 which connects with an internal taper 878 formed on the plug 850. The plug 850 includes a planar support surface 836 which, together with arcuate surface 834 of the body 820, form support surface 826 of the prosthesis 810 for the securing the prosthesis 810 to the humerus 12.

Referring now to FIGURE 13, another embodiment of the present invention is shown as kit 900. The kit 900 includes a body 920 similar to the body 720 of the prosthesis 710 of FIGURE 11. The body 920 includes an articulating surface 922 and an opposed support surface 934. The kit 900 further includes a first spacer in the form of a plug 950. The first spacer 950 is similar to the first spacer or plug 750 of the prosthesis 710 of FIGURE 11. The body 920 and the first spacer 950 combine to form prosthetic member 910.

The prosthetic member 910 may further include an optional stem 940 similar to the stem 740 of FIGURE 11. The kit 910 in addition to the first spacer 950 includes a second spacer 980. The second spacer 980 may selectively be included or excluded from the prosthetic member 910 such that planar support surface 936 may be located for example on the first spacer 950 or alternatively on the second spacer 980. The kit 900 may optionally further include a third spacer 982 or additional spacers (not shown). When the kit 900 includes the body 920, the first spacer 950 and the second spacer 980, the kit 900 may be utilized by selectively picking the inclusion or non-inclusion of the second spacer 980, thereby providing for a variation in the location of the support surface 936. The kit 900 permits the use of a prosthesis with a variety of locations for the support surface 936. The ability to vary the location of the support surface is important when dealing with diseased humerus in which the flattened head may vary from patient to patient, and the corresponding required amount of resection may vary for a given geometry of the humerus.

The prosthesis 910 may be built by utilizing the body 920 and the plug 950 as well as a combination of one or the other of the second and third spacers 980 or 982, respectively, or by the use of both spacers 980 and 982. Similarly, the prosthetic member 910 may be performed without the use of either the second spacer 980 or the third spacer 982.

Preferably and as shown in FIGURE 13, the first spacer 950 is secured to the body 920 by use of a first tapered connection 956. The first tapered connection 956 as shown in FIGURE 13, includes an external taper 960 formed on the first spacer 950, which mates with an internal taper 962 formed on the body 920. The second spacer 980 may be secured to the plug 950 by the use of a second tapered connection 974. The second tapered connection 974 may include an external taper 976 formed on the second spacer 980 which mates with an internal taper 978 formed in the first spacer 950. Similarly, the second spacer 980 may be connected to the third spacer 982 by means of a third tapered connection 984. Similarly, the third spacer 982 may be connected to the stem 940 by means of a fourth tapered connection 986. Preferably and as shown in FIGURE 13, the second tapered connection 974, the third tapered connection 984 and the fourth tapered connection 986 are identical to each other so that the stem 940 may be connected to any of the first spacer 950, second spacer (980) or third spacer 982.

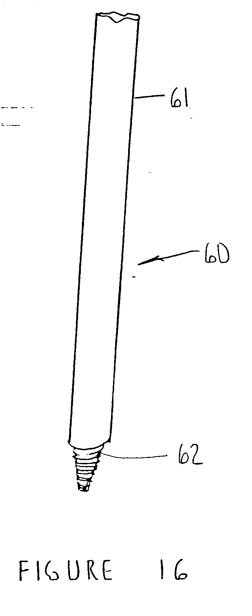

Referring now to FIGURE 14, another embodiment of the present invention in the form of kit 1000 is shown. Kit 1000 is similar to kit 900 of FIGURE 13 but includes additional components so that patients with greatly varying humeral sizes as well as varying conditions of the flattening of the humeral head may be accommodated within the kit 1000. For example, as shown in FIGURE 14, the kit 1000 includes a plurality of cups, plugs, spacers and stems so that a wide variety of patient humeral conditions can be accommodated. As shown in FIGURE 14, the kit 1000 includes a first cup 1020 having a first size articulating surface 1022. The kit 1000 also includes a second cup 1020A. The cup 1020A includes an articulating surface 1022A, which is larger than the articulating surface 1022. The kit 1000 may also include a third cup 1020B, having an articulating surface 1022B, which is larger than the articulating surface 1022A of the cup 1020A. So that the cups 1020, 1020A and 1020B may be utilized with common spacers, plugs and stems, preferably and as shown in FIGURE 14, the cup 1020 has an internal arcuate surface 1034 which is the same size and shape as the articulating surface 1034A of the cup 1020A which is also the same size and shape as articulating inner surface 1034B of the cup 1020B.

The kit 1000 further includes a first plug 1050 having a planar surface 1036 and an opposed arcuate surface 1072. The arcuate surface 1072 of the first plug 1050 matingly fits against the arcuate surface 1034 of the first cup 1020. The kit 1000 further includes a second plug 1050A as well as a third plug 1050B. The first plug 1050, the second plug 1050A and the third plug 1050B preferably each have a respective arcuate periphery 1072, 1072A and 1072B which all matingly fit with the arcuate surface 1034 of the cup 1020. Thus, the first plug 1050, the second plug 1050A and the third plug 1050B may be selectively mated with the first cup 1020. The first plug 1050, the second plug 1050A and the third plug 1050B each have a respective support surface 1036, 1036A and 1036B which provide for varying amounts of resection of the humerus 12.

The kit 1000 further includes a first spacer 1080, a second spacer 1080A, and a third spacer 1080B and a fourth spacer 1080C. Each of the spacers 1080, 1080A, 1080B and 1080C has a different thickness to accommodate a different amount of resection of the humerus 12.

The kit 1000 may further include a plurality of stems, for example, a first stem 1040, a second stem 1040A, and a third stem 1040B. Each of the stems 1040, 1040A and 1040B has a different length to accommodate a different size humerus. Preferably, and as shown in FIGURE 14, for the components of the kit 1000 to be able to be easily matched, the components have external tapers 1060 which are all identical as well as internal tapers 1062 which are all identical, so that any internal taper 1062 may fit against an external taper 1060.

For example, as shown in FIGURE 14, the cup 1020 may be combined with the plug 1050 to form a first prosthetic member 1010 and the second plug 1050A may be combined with the second cup 1020A to form a second prosthetic member 1011.

The kit 1000 may further include instruments 1051 to be used in conjunction with installing and removing the prosthesis.

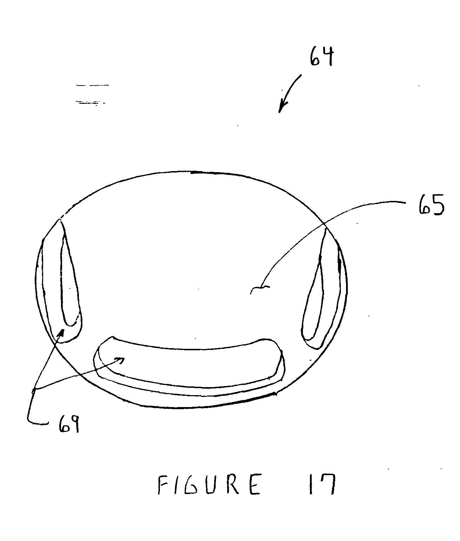

Referring now to FIGURE 15, a gauge 51 is shown for use in determining the amount of resection required to the flattened humeral head and the corresponding spacer or spacers required for use with the prosthesis of the present invention. The gauge 51 includes a gauge body 52 including an arcuate contact surface 53, which has a shape similar to that of the interior of the prosthesis to be implanted. A rod 54 is slidably fitted within a longitudinal opening 55 in the gauge body 52. A contact probe 56 is positioned on an end of the rod 54. The contact probe 56 contacts the flattened humeral head 57. The position of the contact probe 56 when in contact with the humeral head 57 is measured at a window 58 in the gauge body 52 and indicia 59 on the rod 54 indicate the appropriate amount of resection of the flattened humeral head 57 and the corresponding spacer required because of the resection.

Referring now to FIGURE 16, a guide pin 60 is shown. The guide pin 60 is utilized in shoulder arthroplasty to guide the resection tool in resecting the humeral head for preparation of the implant of the prosthesis of the present invention. The guide pin 60 includes a cylindrical body 61, as well as a cutting edge 62. Guide pins 60 are commercially available from, for example, the assignee of the present invention.

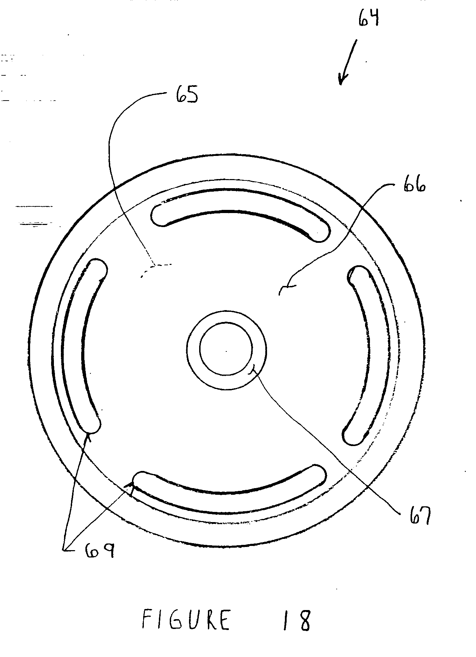

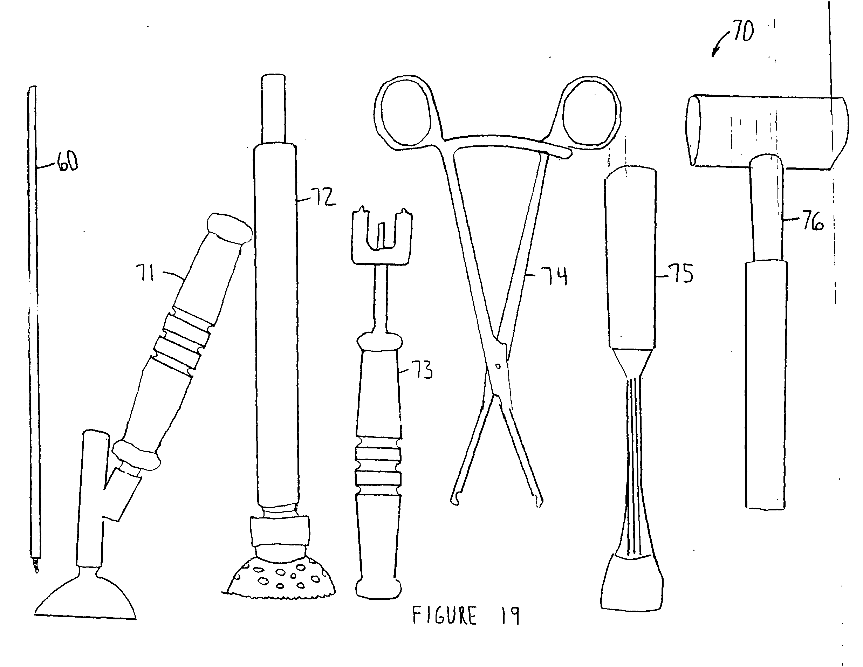

Referring now to FIGURES 17 and 18, a trial 64 for use with the prosthesis of the present invention is shown. The trial 64 is utilized during shoulder arthroplasty to verify the proper selection of the prosthetic member by implanting the trial 64 into the humeral head and performing trial reductions on the arm to verify the selection of the particularly sized trial and corresponding prosthesis. The trial 64 is removed and replaced with the corresponding prosthesis. The trial 64 may be reused after sterilization. The trial is made of any suitable durable material and may, for example, be made of a durable plastic that may be sterilized by standard methods such as used in an autoclave.

The trial 64 mimics the size and shape of the prosthesis. The trial 64 therefore includes an articulating surface 65 and an opposed support surface 66. The trial 64 further includes a stem 67 extending outwardly from the support surface 66. As shown in FIGURES 17 and 18, the trial 64 may also include a plurality of spaced apart openings 69 to assist in the removal of the trial 64.

Referring now to FIGURE 19, a kit 70 for use when performing an arthroplasty to implant the prosthesis of the present invention. The kit 70 includes the guide pin 60, a guide pin alignment tool 71 for assisting in aligning the guide pin and positioning it into the humerus. The instrument kit 70 also includes a cutting assembly tool 72 for preparing the humeral head. The instrument kit 70 further includes a cutting tool assembly wrench 73 for assembling and disassembling the cutting tool from the cutting tool assembly 72. The instrument kit 70 also includes forceps 74 for securely gripping items. The instrument kit 70 also includes a humeral head impactor 75, which is used with a surgical mallet 76 to impact the implant into its full seat.

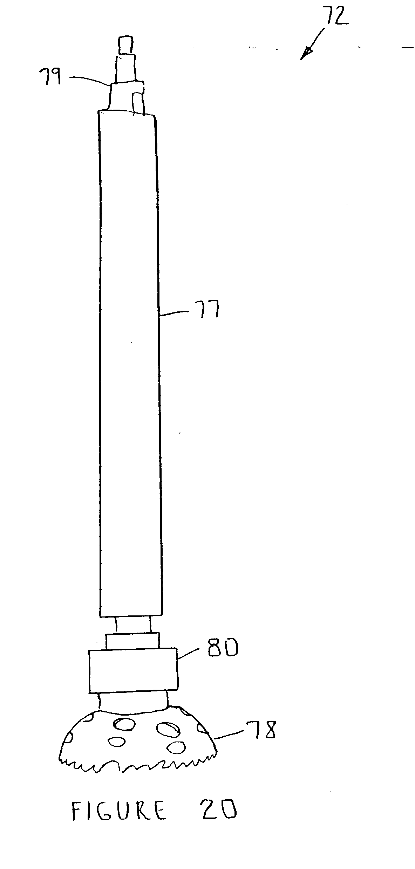

Referring now to FIGURE 20, the cutting tool assembly 72 is shown in greater detail. The cutting tool assembly 72 includes a tool holder 77 to which a cutting tool 78 in the form of, for example, a hemispherically shaped reamer is attached. The tool holder 77 includes a drive adapter 79 for attaching a power device (not shown) to the cutting tool assembly 72. The tool holder 77 further includes an adapter 80 for securing the cutting tool 78 to the tool holder 77.

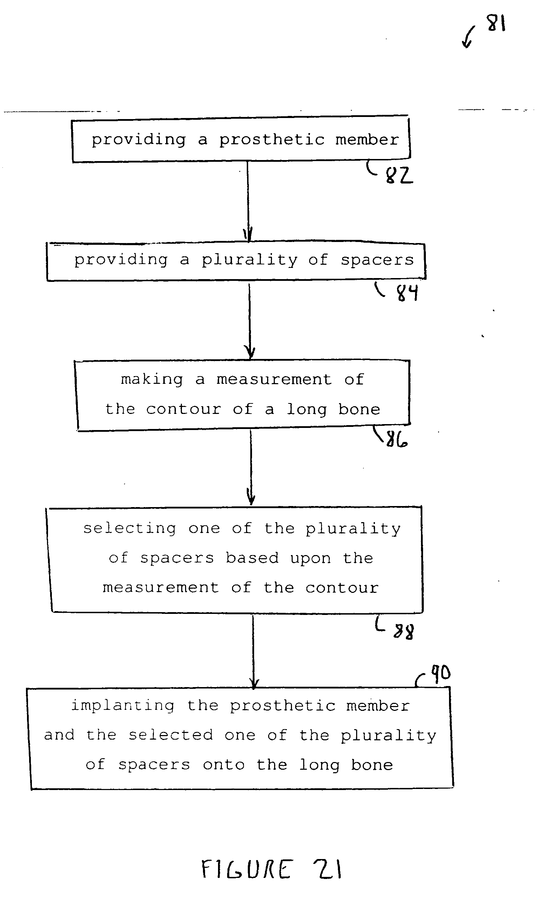

Referring now to FIGURE 21, a further embodiment of the present invention is shown in a surgical method for providing joint arthroplasty 81. The method 81 includes a first step 82 of providing a prosthetic member. The method 81 also includes a second step 84 of providing a plurality of spacers and a third step 86 of making a measurement of the contour of a long bone. The method 81 further includes a fourth step 88 of selecting one of the plurality of spacers based upon the measurement of the contour. The method 81 further includes a fifth step 90 of implanting the prosthetic member and the selected one of the plurality of spacers onto the long bone.

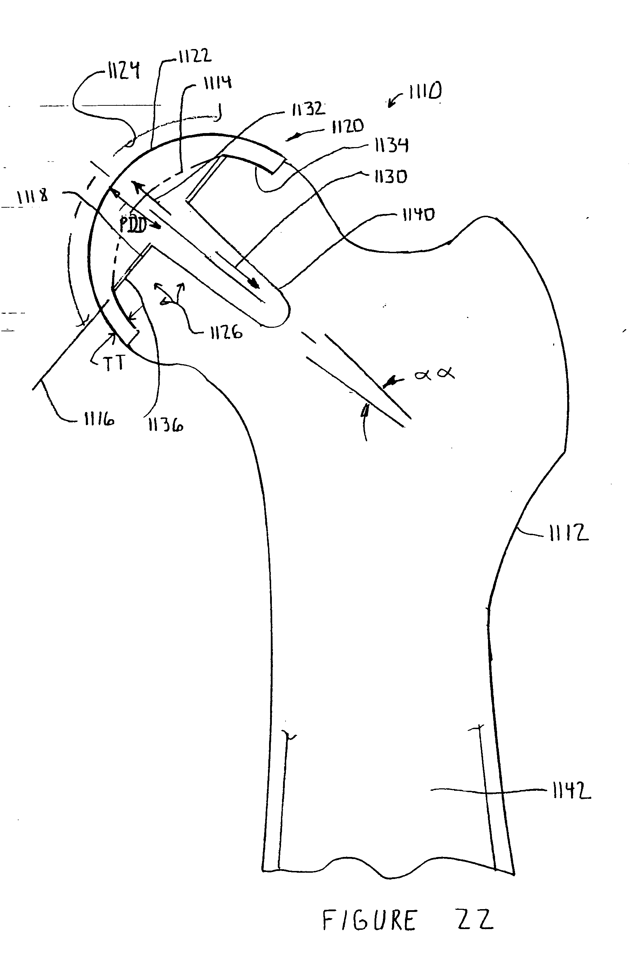

Referring now to FIGURE 22, a prosthesis 1110 according to the present invention is shown. The prosthesis 1110 is used in performing hip joint arthroplasty. As shown in FIGURE 22, the prosthesis 1110 is shown for use with a femur 1112.

Typically, when the prosthesis 1110 is required on a femur 1112, the femur 1112 includes a flattened femoral head forming a bony defect 1114. Preferably and as shown in FIGURE 22, the femur 1112 is resected along resection plane 1116 providing a prepared surface 1118.

The prosthesis 1110 is similar to the prosthesis 10 of FIGURE 1, except the prosthesis 1110 is adapted to be used on the head of femur 1112. The prosthesis 1110 includes a body 1120. The body 1120 has an articulating surface 1122, which is in a combination of mostly rolling and sliding contact with acetabulum 1124. The body 1120 also includes a support surface 1126 opposed to the articulating surface 1122. As can be seen in FIGURE 22, the support surface 1126 is located in a first direction 1130 opposed to the second direction 1132 of the articulating surface 1122.

The prosthesis 1110 may have any suitable size and shape capable of providing the articulating surface 1122 for cooperation with the acetabulum 1124 and to provide the intimate contact with the resected surface 1118 of the femur 1112.

As shown in FIGURE 22, the body 1120 may have a shape such that articulating surface 1122 is convex. Opposed from the articulating surface 1122 may be an arcuate support surface 1134. The arcuate support surface 1134 may be concave. For example, the arcuate support surface 1134 and the articulating surface 1122 may form a sector of a hollow sphere having a thickness TT and with the articulating surface 1122 being defined by a radius and the arcuate support surface 1134 being defined by a radius.

For simplicity and as shown in FIGURE 22, the support surface 1126 may include a planar portion 1136 which, depending on the position of the resected plane 1116, may be defined by a plane dimension PDD. The planar surface 1136 and the arcuate support surface 1134 define the support surface 1126. With the planar support surface 1136 and the resected surface 1118, both being planar, the resected surface 1118 and the planar portion 1136 provide intimate contact between the prosthesis 1110 and the femur 1112.

For sufficient securement of the prosthesis 1110 to the femur 1112, the prosthesis 1110 may further include a stem 1140 for securement with cancellous bone 1142 of the femur 1112. The stem 1140 may have any suitable size and shape capable of securing the prosthesis 1110 to the femur 1112. For example, the stem 1140 may have a generally cylindrical shape and may have a length extending distally from the planar portion 1136 in the first direction 1130. The stem 1140, as shown in FIGURE 22, may have a slight taper defined by angle αα. For example, the angle *αα* may be 3 to 20°.

As shown in FIGURE 22, the prosthesis 1110 may be integral or made from a single piece. The prosthesis 1110 may be made of any suitable durable material that is compatible with the human anatomy and provides sufficient strength and wear properties. For example, the prosthesis 1110 may be made of a durable plastic, a ceramic or a metal. For durability and strength, the prosthesis 1110 is preferably made of a metal. For example, the prosthesis 1110 may be made of a titanium alloy, a cobalt chromium alloy, or stainless steel.

The prosthesis 1110 may be made by any suitable process. For example, the prosthesis 1110 may be machined from bar stock, forged, cast or made from a material such as that available from Liquidmetals.

## Claims

1. A prosthesis for use in performing joint arthroplasty, said prosthesis to be fitted to a long bone, said prosthesis comprising a body including an articulating surface and a support surface opposed to the articulating surface, the support surface adapted for intimate contact with a prepared surface of the long bone.

2. The prosthesis of claim 1, wherein said body includes a spacer, said spacer including the support surface.

3. The prosthesis of claim 1, wherein at least a portion of the support surface is generally planar.

4. The prosthesis of claim 1, further comprising a stem extendable from the body in a direction opposed to the articulating surface.

5. The prosthesis of claim 4, wherein said stem is integral with said body.

6. The prosthesis of claim 2, wherein at least one of said body and said spacer comprise a connector for connecting said spacer to said body.

7. The prosthesis of claim 6, wherein the connector comprises at least one of a threaded connection, a press-fit connection, a tapered connection, and a threaded fastener.

8. The prosthesis of claim 1, wherein said body includes a portion thereof having a coating to encourage bone ingrowth.

9. The prosthesis of claim 1, in which the body includes a hemispherical cup and a plug having a portion conforming to the inner periphery of the cup; and which also includes a stem connected to the plug.

10. A kit for use in performing joint arthroplasty on a bone, said kit comprising:
a prosthetic member including an articulating surface and a support surface opposed to the articulating surface;
a first spacer positionable between the long bone and the support surface of said prosthetic member; and
a second spacer positionable between the long bone and the support surface of said prosthetic member, whereby said prosthetic member and at least one of said first spacer and said second spacer may be used selectively used to form a properly sized prosthesis to perform the joint arthroplasty.

11. The kit of claim 10, further comprising a second prosthetic member including a second prosthetic member articulating surface and a second prosthetic member support surface opposed to the second prosthetic member articulating surface.

12. The kit of claim 10, which includes a gauge having a gauge body with a gauge contact portion thereof for contact with the bone, the gauge contact portion being shaped to correspond to the contact surface of said prosthetic member.

13. The kit of claim 10, which includes a second prosthetic member having a second support surface for contact with the bone, said second prosthetic member having at least one dimension different from the corresponding dimension of said first mentioned prosthetic member;
a first gauge, said first gauge including a first gauge body having a first gauge contact portion thereof for contact with the bone, the first gauge contact portion being shaped to correspond to the support surface of said first mentioned prosthetic member; and
a second gauge, said second gauge including a second gauge body having a second gauge contact portion thereof for contact with the bone, the second gauge contact portion being shaped to correspond to the support surface of said second prosthetic member.

14. The kit of claim 10, wherein said prosthetic member includes a hemispherical cup and a plug having a portion conforming to the inner periphery of the cup.

15. The kit of claim 14, further comprising at least one of a second hemispherical cup and a second plug.

16. The kit of claim 10, further comprising a stem extendable from said prosthetic member in a direction opposed to the articulating surface.

17. The kit of claim 16, further comprising a second stem.

18. The kit of claim 10, wherein at least one of said prosthetic member, said first spacer and said second spacer comprise a connector for connecting one of said first spacer and said second spacer to said prosthetic member.

19. The kit of claim 18, wherein said connector comprises at least one of a threaded connection, a press-fit connection, a tapered connection, and a threaded fastener.
